(19) Europäisches Patentamt
European Patent Office
Office européen des brevets

(11) Numéro de publication : **0 491 594 A1**

(12) # DEMANDE DE BREVET EUROPEEN

(21) Numéro de dépôt : **91403291.7**

(51) Int. Cl.$^5$ : **A61K 49/00**

(22) Date de dépôt : **05.12.91**

(30) Priorité : **06.12.90 FR 9015292**

(43) Date de publication de la demande :
**24.06.92 Bulletin 92/26**

(84) Etats contractants désignés :
**AT BE CH DE DK ES FR GB GR IT LI LU NL SE**

(71) Demandeur : **MEDGENIX GROUP, S.A.**
**1, place d'Italie**
**B-4020 Liège (BE)**

(72) Inventeur : **Chiu, Kwok Wai**
**High Street 152**
**Gloucester GL1 4TF (GB)**
Inventeur : **Thornback, John R., Dr.**
**11 rue des papeteries**
**B-1325 Chaumont Gistoux (BE)**

(74) Mandataire : **Warcoin, Jacques et al**
**Cabinet Régimbeau 26, avenue Kléber**
**F-75116 Paris (FR)**

(54) **Agent de contraste constitué par un complexe neutre d'un cation paramagnétique et ligand pour former un complexe.**

(57) La présente invention a pour objet un agent de contraste pour l'imagerie par RMN constitué d'un complexe de coordination neutre entre un ligand et un cation métallique, ou un hydrate de ce complexe, de formule :

$$ML.\ mH_2O \qquad (I)$$

dans laquelle
— M désigne un cation métallique paramagnétique trivalent
— m est un entier de 0 à 5
— L est un ligand de formule :

$$A\text{-}[C(R_1,R_2)\text{-}(C(R_8^i,R_9^i))_n\text{-}C(R_3,R_4)\text{-}N{=}C(R_5)\text{-}C(R_6){=}C(R_7)\text{-}O^-]_3 \qquad (II)$$

dans laquelle
— A représente N ou C-$R_{10}$,
— $R_1$ à $R_{10}$, identiques ou différents, sont choisis parmi H, un hydroxyle, un halogène, un groupe alkyle, alcoxy, aryle, alkenyle, cycloalkyle groupes éventuellement substitués par un ou des groupes halogène, hydroxyle, alkyle, alcoxy, nitrile, nitro, amine, ou hydroxyalkyle.
— n est un entier de 0 à 3, i est un entier de 1 à n.

La présente invention a également pour objet un ligand pouvant former des complexes de coordination avec des cations métalliques trivalents, caractérisé en ce qu'il répond à la formule :

$$A\text{-}[C(R_1,R_2)\text{-}(C(R_8^i,R_9^i))_n\text{-}C(R_3,R_4)\text{-}NH\text{-}C(R_6){=}C(R_6)\text{-}C(R_7){=}O]_3 \qquad (III)$$

et un procédé de préparation de ces complexes.

EP 0 491 594 A1

L'invention concerne de nouveaux agents de contraste utilisables dans le diagnostic médical par imagerie par résonance magnétique (IRM).

La résonance magnétique nucléaire est maintenant largement utilisée pour obtenir des images spatiales de sujets humains en vue de l'établissement de diagnostics cliniques.

Par cette technique, on obtient in vivo une image d'un organe ou d'un tissu en plaçant le sujet dans un champ magnétique externe fort et en observant les effets de ce champ sur les propriétés magnétiques des protons notamment du proton de l'eau, contenus dans, et aux environs desdits organes ou desdits tissus.

Une fois que la source d'énergie externe est supprimée, les noyaux à l'état excité tendent à revenir à un état d'énergie plus stable. Le retour du noyau à l'équilibre est caractérisé par deux temps de relaxation appelés T1 et T2. Ces deux paramètres dépendent de l'environnement physique et chimique de l'eau de l'organe ou du tissu, et ils décrivent le retour à l'équilibre du magnétisme nucléaire des noyaux de l'échantillon dans les directions parallèle ou perpendiculaire aux champs magnétiques appliqués. Ainsi, T1 représente une constante de temps qui décrit un mode de dissipation de l'énergie, en particulier la perte d'énergie vers l'environnement moléculaire local, c'est-à-dire le réseau. C'est par l'étude des temps de relaxation des protons des molécules présentes, en particulier des protons de l'eau, qu'il est possible de produire, par des calculs relativement complexes, une image visuelle de la structure du tissu concerné.

L'image produite, toutefois, manque souvent de définition et de clarté, à cause de la similitude des signaux d'autres tissus. Pour éviter cet inconvénient, on fait appel à des agents de contraste. Ce sont des substances qui ont un effet sur les paramètres RMN (temps de relaxation $T_1$, $T_2$ et relaxivité) de diverses espèces chimiques présentes autour d'eux.

En tant qu'agents de contraste, on a déjà utilisé des produits paramagnétiques, qui contiennent des électrons non appareillés qui n'intéragissent pas et ne sont pas couplés. Il s'agit par exemple d'ions en solution. Ainsi, la publication Européenne de brevets EP 071 564 décrit l'utilisation, pour le diagnostic par IRM chez l'homme, d'ions $Gd^{3+}$ chélatés avec de l'acide pentaacétique diéthylène triamine (DTPA). Le chélate ainsi formé permet de diminuer le temps de relaxation T1 et d'augmenter ainsi le contraste et la précision de l'image. L'ion $Gd^{3+}$ étant lui-même toxique, il est nécessaire de le chélater pour une utilisation in vivo. Ce composé a été utilisé par voie intraveineuse pour démontrer des altérations pathologiques de "blood brain barrier" (R.C. BRASCH et al American Journal of ROENTGENOLOGY, 142, page 625, 630 - 1984). Toutefois, ce produit n'est pas adapté à d'autres utilisations diagnostiques. Par exemple, dans le foie, il est absorbé de la même façon par les cellules normales et les cellules cancéreuses et on ne peut donc distinguer les tissus sains des tissus altérés.

L'invention vise donc à fournir de nouveaux agents de contraste utilisables en IRM qui présentent par rapport aux agents connus, une plus grande spécificité tissulaire.

Un but de la présente invention est de proposer un agent de contraste du type complexe comme paramagnétique soluble en solution aqueuse pouvant être administré par voie parentérale, par exemple par injection intraveineuse, de façon à obtenir un effet de contraste en imagerie par RMN d'un organe spécifique.

Plus précisément, un but de la présente invention est de proposer un agent de contraste qui, après administration à faible dose, conduit à un accroissement spécifique de la relaxation du proton de l'eau dans un organe particulier sans interférer avec d'autres organes et sans causer d'effets secondaires.

Un autre but est que l'agent de contraste présente à la fois une haute stabilité, une faible toxicité, c'est-à-dire soit physiologiquement tolérable d'une part, et que d'autre il présente les propriétés de relaxivité et d'osmolarité satisfaisantes pour une bonne imagerie par contraste.

On a maintenant constaté, de façon surprenante, que des composés $LH_3$ répondant à la formule développée suivante :

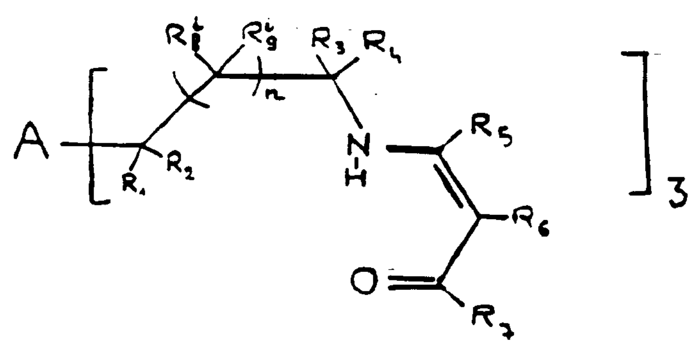

permettent la formation de complexes neutres d'ions métalliques paramagnétiques trivalent qui présentent une

spécificité tissulaire accrue lors de leur utilisation en tant qu'agents de contraste en IRM.

C'est pourquoi l'invention a pour objet un agent de contraste pour l'imagerie par RMN constitué d'un complexe de coordination neutre entre un ligand et un cation métallique ou un hydrate de ce complexe, de formule :

$$ML.\ mH_2O \qquad (I)$$

dans laquelle

– M désigne un cation métallique paramagnétique trivalent

– m est un entier de 0 à 5

– L est un ligand de formule :

$$A\text{-}[C(R_1,R_2)\text{-}(C(R_8^i,R_9^i)_n)\text{-}C(R_3,R_4)\text{-}N{=}C(R_5)\text{-}C(R_6){=}C(R_7)\text{-}O^-]_3 \qquad (II)$$

(correspondant à la forme déprotonée du ligand $LH_3$, étant de formule :

$$A[C(R_1,R_2)\text{-}(C(R_8^i\ ,R_9^i)_n)\text{-}C(R_3,R_4)\text{-}NH\text{-}C(R_5){=}C(R_5)\text{-}C(R_7){=}O]_3)$$

dans laquelle

– A représente N ou C-$R_{10}$,

– R $_1$ à $R_{10}$, identiques ou différents, sont choisis parmi H, un hydroxyle, un halogène, un groupe alkyle, alcoxy, aryle, alkenyle, cycloalkyle groupes éventuellement substitués par un ou des groupes halogène, hydroxyle, alkyle, alcoxy nitrile, nitro, amine ou hydroxyalkyle

– n est un entier allant de 0 à 3, i est un entier de 1 à n.

En fait, compte tenu des spectres IR obtenus, il semble qu'il y ait une décocalisation des doubles liaisons et une coordination de complexation que l'on peut schématiser comme suit:

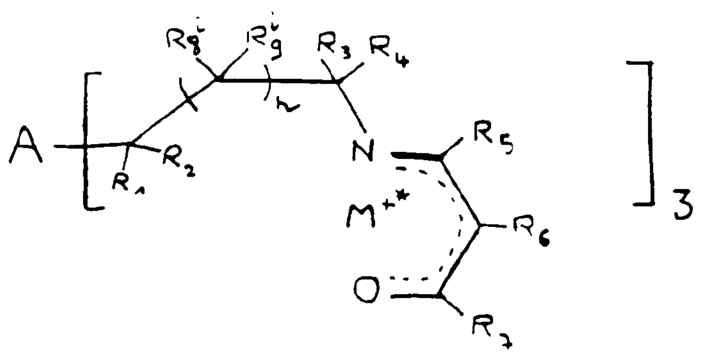

$* (+) = \frac{1}{3} (3+)$

qui est peut être à l'origine de la grande stabilité du complexe.

Ces complexes entre le ligand et le cation paramagnétique sont neutres. Ils se dissolvent très facilement dans l'eau à PH physiologique.

Selon la présente invention, on entend par alkyle ou alcoxy des radicaux en $C_1$ à $C_7$ de préférence en $C_1$ à $C_3$ et aryle se réfère à un phényle éventuellement substitué. Le terme alkenyle se réfère à un groupe en $C_2$-$C_7$. Le terme cycloalkyle se réfère ici à un groupe en $C_5$-$C_7$.

On citera en particulier les agents de contraste pour lesquels A représente N, $R_1$ à $R_9$ représente H, un halogène, un alcoxy en $C_1$ à $C_3$, un radical alkyle en $C_1$ à $C_3$ éventuellement substitués par un halogène, un hydroxy, un alcoxy en $C_1$-$C_2$.

Dans un mode de réalisation particulièrement approprié dans la formule (II) ou (III) n = 0.

Les agents de contraste de l'invention sont particulièrement efficaces lorsque le métal paramagnétique est choisi parmi les ions divalents ou trivalents de métaux de transition ou de lanthanides paramagnétiques.

On peut citer plus particulièrement les ions suivants :

$Mn^{3+}$, $Cr^{3+}$, $Fe^{3+}$, $Gd^{3+}$, $Dy^{3+}$, $Ho^{3+}$ de préférence $Gd^{3+}$

On citera également plus spécialement l'agent de formule :

$$Gd^{(3+)}[N(CH_2CH_2\text{-}N{=}C(CH_3)\text{-}CH{=}C(CH_3)\text{-}O^-)_3]$$

Enfin la présente invention a également pour objet un procédé de préparation des agents de contraste selon l'invention que l'on effectue dans des conditions réactionnelles connues de l'homme de l'Art [1,2], caractérisé en ce que l'on fait réagir un sel de formule $MX_3$ dans laquelle X est un mono anion provenant d'un acide HX avec un ligand $LH_3$ de formule :

$$A\text{-}[C(R_1,R_2)\text{-}(C(R_8^i,R_9^i)_n)\text{-}C(R_3,R_4)\text{-}NH\text{-}C(R_5){=}C(R_6)\text{-}C(R_7){=}O]_3$$

On peut citer comme anion monovalent, ceux des halogènes, les groupes carboxylates ($RCOO^-$) ou nitrates ($NO^-_3$) provenant respectivement des acides carboxylique et nitrique.

La présente invention a également pour objet un ligand pouvant former des complexes de coordination

avec des cations métalliques trivalents, caractérisé en ce qu'il répond à la formule :

$$A\text{-}[C(R_1,R_2)\text{-}(C(R^i_8,R^i_9)_n)\text{-}C(R_3,R_4)\text{-}NH\text{-}C(R_5)=C(R_6)\text{-}C(R_7)=O]_3 \quad (III)$$

dans laquelle A, $R_1$, $R_{10}$ et n ont les significations données précédemment.

Ces ligands peuvent être utilisés également pour complexer d'autres métaux cationiques trivalents autres que paramagnétiques pour des utilisations autres que comme agent de contraste.

Ces ligands peuvent être obtenus selon des procédés connus de l'homme de l'Art. Par exemple on fait réagir un composé de formule:

$$A\text{-}[C(R_1,R_2)\text{-}(C(R^i_8,R^i_9)_n)\text{-}C(R_3,R_4)\text{-}NH_2]_3 \quad (III)b$$

avec 3 diones de formule

$$O=(R_5)C\text{-}CH(R_6)\text{-}C(R_7)=O \quad (IV)b$$

dans des conditions réactionnelles connues de l'homme de l'Art [3,4].

D'autres avantages et caractéristiques de l'invention apparaîtront à la lumière des exemples qui vont suivre.

Dans l'ensemble des exemples, le temps de relaxation T1 est mesuré à l'aide du Minispec Brücker, 20 MHz à 37°C.

Tous les réactifs chimiques, sauf indication contraire, sont couramment disponibles dans le commerce et utilisés tels quels sans étape ultérieure de purification.

Les spectres infra-rouges sont enregistrés sur un appareil PE 297. Les spectres RMN sont enregistrés sur un appareil Jeol 6 x 270 (270 MHz) et les spectres de masse sont enregistrés sur un appareil Kratos MS80 en utilisant la spectrométrie Fab à l'argon.

Les figures 1 et 2 représentent les spectres IR du ligand $N[CH_2CH_2NH\text{-}C(CH_3)=CH\text{-}C(CH_3)=O]_3$ et du complexe $Gd[N[CH_2CH_2NC\ (CH_3)CHC(CH_3)O]_3]$ respectivement.

**<u>EXEMPLE 1</u>: Préparation du ligand $N[CH_2CH_2NH\text{-}C(CH_3)=CH\text{-}C(CH_3)=O]_3$**

A une solution de tris(2-aminoéthyl)amine (1,46 g, 1,49 ml, 10 mmol) dans l'acétonitrile (20 ml) on ajoute du 2,4-pentanedione (3,6 ml, 36 mmol). Une solution orange se forme instantanément. Le mélange réactionnel est agité à 20°C pendant 72 heures. Les matières volatiles sont éliminées sous vide pour donner une huile orange. On ajoute de l'eau, la solution aqueuse est extraite avec du $CH_2Cl_2$ , la solution de $CH_2Cl_2$ est évaporée pour donner une huile orange. Le composé est purifié par Flash Column Chromatography, (Silica gel 60, Merck) en utilisant premièrement un mélange de EtOAc: MeOH: $NEt_2H$ (97,5 : 2 : 0,5), puis avec un mélange EtOAc : MeOH : $NEt_2H$ (96 : 3 : 0,5) pour éluer le produit. Le rendement global est de 76 % (3,0 g).

Données analytiques du produit obtenu :

– Mass EI / 70 cv $M^+$ m/z = 393
– I.R/$cm^{-1}$ = 3430 (brm), 3046 (w), 1630 (vs), 1558 (vs), 1510 (s), 1440 (m), 1370 (m), 1300 (s), 1080 (w), 1010 (w), 970 (w), 889 (w), 720 (m), 630 (w) (spectre de la figure 1)
– $^1$Hnmr, $d^6$ DM50 / $\delta$ ppm :

1,92, 2,04 <u>singulets</u>, 18 H, C<u>H</u>$_3$
2,67, 3,46 <u>triplets</u>, 12 H, C<u>H</u>$_2$ $^3J_{HH}$ = 6,5 Hz
4,94 <u>singulet</u> 3 H, C<u>H</u>
10,8 <u>(br)singulet</u> 3 H, N<u>H</u>

**<u>EXEMPLE 2</u>: Préparation du complexe $Gd[N[CH_2CH_2NC(CH_3)CHC(CH_3)O]_3]$**

A une solution $GdCl_3$ (0,4g ; Immol) dans l'éthanol absolu (10 $cm^3$) on ajoute le ligand $N[CH_2CH_2\text{-}NH\text{-}C(CH_3)=CH\text{-}C(CH_3)=O]_3$ (0,84g ; 2,14 mmol en solutions dans 5 ml d'éthanol) et le mélange est porté à reflux pendant 20 minutes puis encore 1,5 heure. La solution turbide est conservée au réfrigérateur pendant environ 66 heures. Un solide blanc enpetite quantité est filtré et le filtrat jaune orangé est séché par évaporation jusqu'à donner une huile que l'on dissout dans l'éthanol absolu. L'addition de diethylether donne un solide orangé pâle qui est filtré et lavé avec du diethylether (5x3 $cm^3$) puis séché sous vide pendant 2 heures. On obtient 0,338 g de produit avec un rendement réactionnel global de 55%.

L'analyse donne les résultats suivants :

– calculée : C, 40,76 ; H, 6,87 ; N, 9,05 %
– trouvée : C, 40,18 ; H, 7,41 ; N, 9,96 %.

Spectre de masse M/Z = 548 $(M\text{-}H)^+$
I.R. = 12 pics à 3399.9, 1606.4, 1542.8, 1437.7, 1363.1, 1299.4, 1211.4, 1095.2, 1018.6, 950.2, 759.2, 655.1 -(spectre IR sur la figure 2)

**EXEMPLE 3 : Résultats**

Les temps de relaxation $T_1$ et $T_2$ et les relaxivités $R_1$ et $R_2$ ont été mesurés pour le complexe de l'exemple 2 :

$$Gd[[N(CH_2CH_2NC(CH_3)CHC(CH_3)O]_3]\text{-}4H_2O$$

Les temps de relaxation $T_1$ et $T_2$ ont été déterminés sur Minispec Brücker à 20 MHz à 37°C. Les relaxivités ont été mesurées sur le minispec 20 MHz. Les mesures ont été effectuées dans l'eau à une concentration de 1 mmol.

$T_1$ = 186 m.sec
$R_1$ = 5,37 $(mM.sec)^{-1}$
$T_2$ = 137 m.sec
$R_2$ = 7,29 $(mM.sec)^{-1}$

**REFERENCES BIBLIOGRAPHIQUES**

(1) N.K. Dutt and P. Bandyopadhyay, P. Sci. Cult., 1961, 27, 403.
(2) J.R. Thornback and G. Wilkinson, J. Chem. Soc. Dalton Trans., 1978, 110.
(3) T. A. Bottomhead and D. J. Robinson,
Aust. J. Chem., 1968, 21, 1365.
(4) N. A. Bailey, D.F. Cook, D. Cummins and E.D. McKenzie, Inorg. Nucl. Chem. Lett., 1975, 11, 51.

**Revendications**

**1)** Agent de contraste pour l'imagerie par RMN constitué d'un complexe de coordination neutre entre un ligand et un cation métallique ou un hydrate de ce complexe, de formule :

$$ML.\ mH_2O \qquad (I)$$

dans laquelle

– M désigne un cation métallique paramagnétique trivalent
– m est un entier de 0 à 5
– L est un ligand de formule :

$$A\text{-}[C(R_1R_2)\text{-}(C(R_8^i,R_8^i)_n)\text{-}C(R_3,R_4)\text{-}N{=}C(R_5)\text{-}C(R_6){=}C(R_7)\text{-}O]_3 \qquad (II)$$

dans laquelle

– A représente N ou C-$R_{10}$,
– $R_1$ à $R_{10}$, identiques ou différents; sont choisis parmi H, un hydroxyle, un halogène, un groupe alkyle, alcoxy, aryle, alkenyle, cycloalkyle, groupes éventuellement substitués par un ou des groupes halogène, hydroxyle, alkyle, alcoxy nitrile, nitro, amine ou hydroxyalkyle,
– n est un entier de 0 à 3 et i est un entier de 1 à n.

**2)** Agent de contraste selon la revendication 1, carctérisé en ce que M représente un cation trivalent de métal de transition ou de canthanide choisi notamment parmi $Cr^{3+}$, $Fe^{3+}$, $Mn^{3+}$, $Dy^{3+}$, $Ho^{3+}$, et de préférence $Gd^{3+}$.

**3)** Agent de contraste selon l'une des revendications 1 ou 2, caractérisé en ce que :

– A représente N
– $R_1$ à $R_9$ représente H, un halogène, un alcoxy en $C_1$ à $C_3$, un radical alkyle en $C_1$ à $C_3$ éventuellement substitués par un halogène, un hydroxy, un alcoxy en $C_1$-$C_2$.

**4)** Agent de contraste selon l'une des revendications 1 à 3, caractérisé en ce que dans la formule II n=0.

**5)** Agent de contraste selon l'une des revendications 1 à 4, caractérisé en ce qu'il s'agit du complexe :

$$Gd^{(3+)}[[N(CH_2CH_2\text{-}N{=}C(CH_3)\text{-}CH{=}C(CH_3\text{-}O^-]_3]$$

**6)** Ligand pouvant former des complexes de coordination avec des cations métalliques trivalents, caractérisé en ce qu'il répond à la formule :

$$A\text{-}[C(R_1,R_2)\text{-}(C(R_8^i,R_8^i)_n)\text{-}C(R_3,R_4)\text{-}NH\text{-}C(R_6){=}C(R_6)\text{-}C(R_7){=}O]_3 \qquad (III)$$

dans laquelle A, n et $R_1$ à $R_{10}$, ont les significations données dans les revendications 1 à 5.

**7)** Procédé de préparation d'un agent de contraste selon l'une des revendications 1 à 5 caractérisé en ce que on fait réagir un sel de formule $MX_3$ dans laquelle X est un monoanion provenant d'un acide HX avec un ligand de formule :

$$A\text{-}[C(R_1,R_2)\text{-}(C(R_8^i,R_8^i)_n)\text{-}C(R_3,R_4)\text{-}NH\text{-}C(R_5){=}C(R_6)\text{-}C(R_7){=}O]_3$$

M, A, $R_1$ à $R_{10}$ , n ayant les significations données dans les revendications 1 à 5.

**8)** Procédé selon la revendication 7 caractérisé en ce que X représente un anion halogène, ou un anion provenant de l'acide nitrique ou d'un acide carboxylique.

**9)** Procédé selon la revendication 7 ou 8, caractérisé en ce que $MX_3$ représente $GdCl_3$.

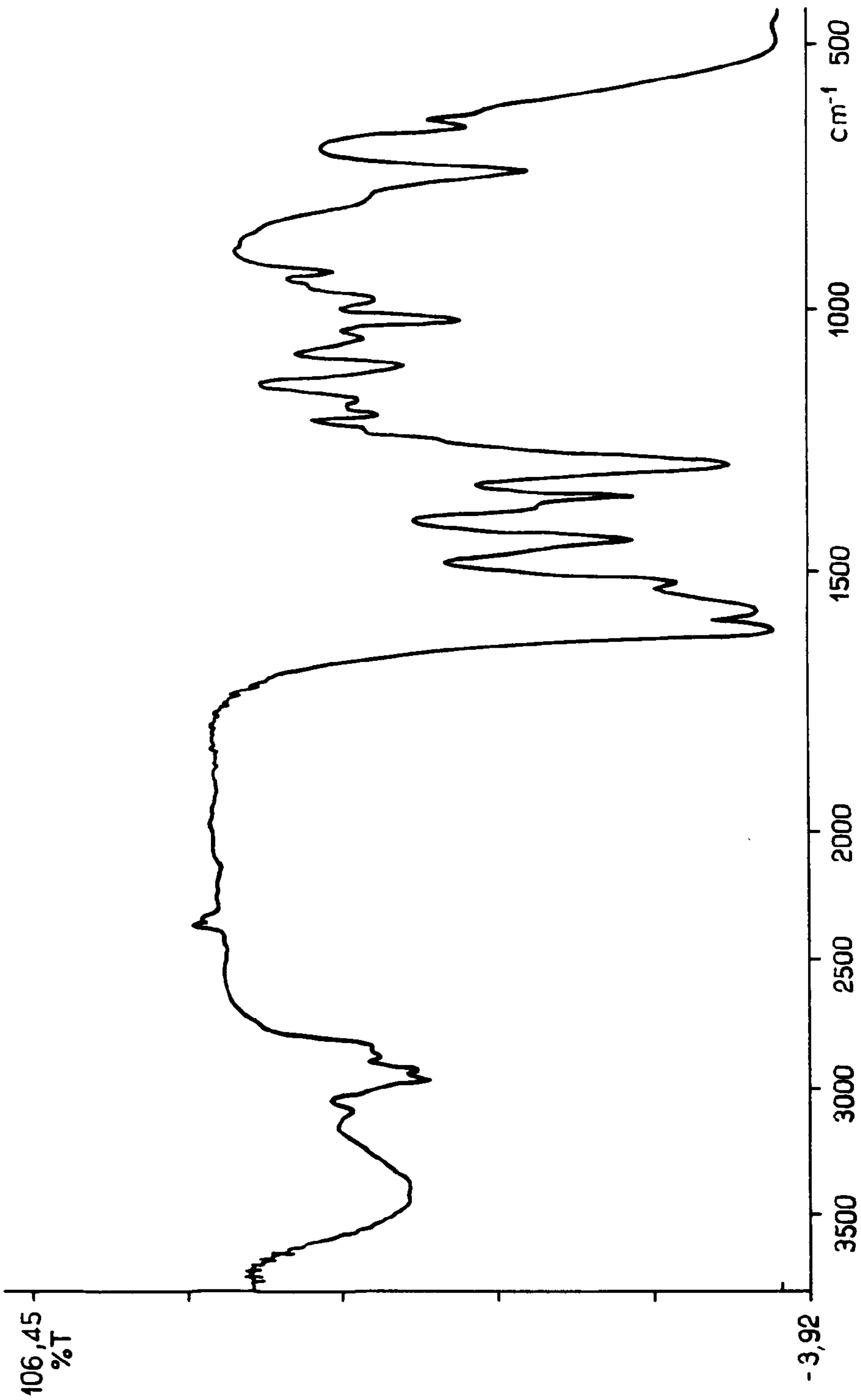


FIG_1

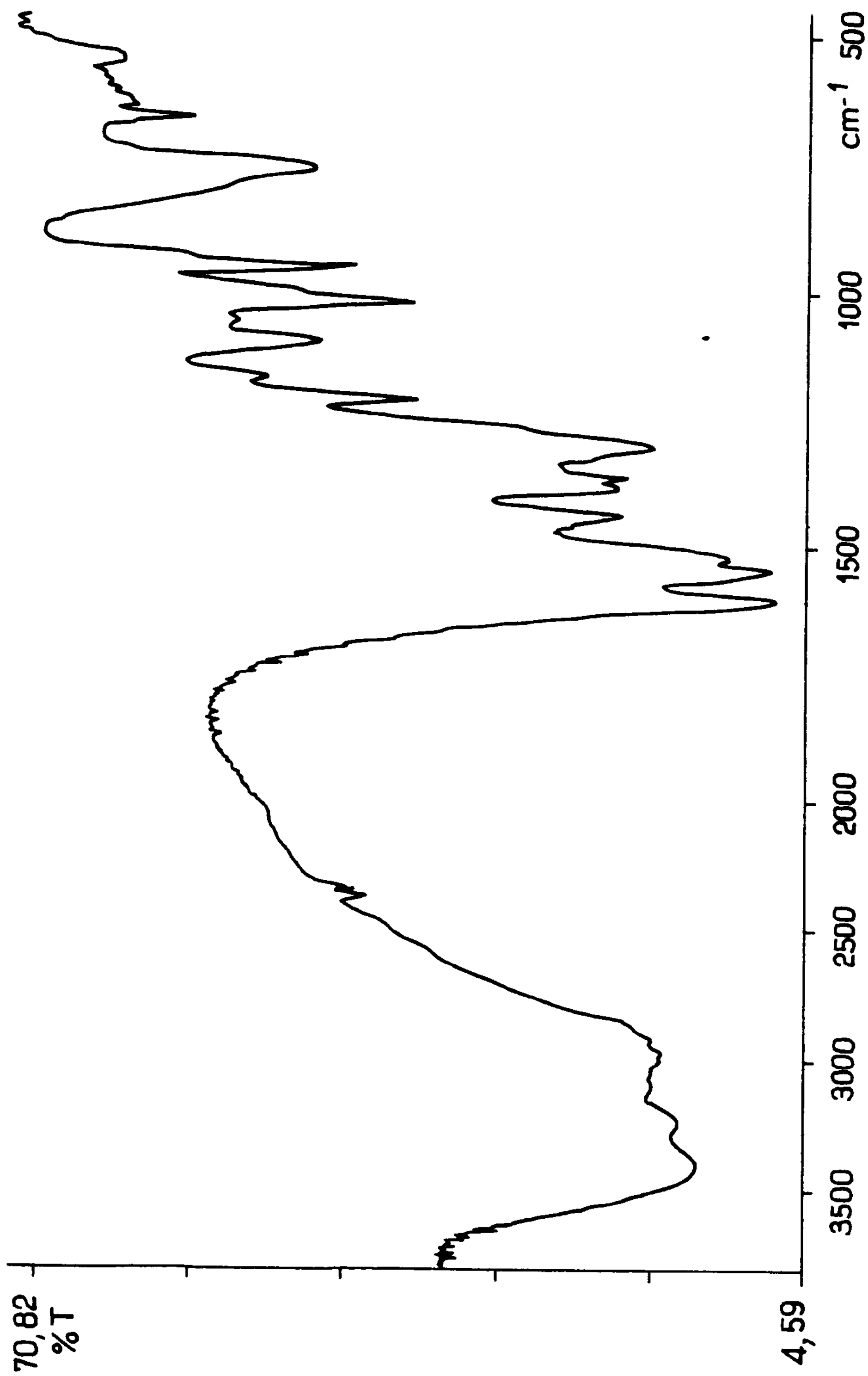


FIG_2

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 91 40 3291

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | FILE SERVER STN KARLSRUHE, FILE CHEMICAL ABSTRACTS, vol. 109, no. 22, abrégé no. 203749t, Columbus, Ohio, US; A. SMITH et al.: "Lanthanide complexes of potentially heptadentate ligands including the structure of (tris(3-aza-4-methylhept-4-ene-6-on-1-yl)amine)tris(nitrato)gadolinium(III), & INORG. CHEM., 27(22), 3929-34<br>* Abrégé *<br>--- | 1-9 | A 61 K 49/00 |
| Y | J. CHEM. SOC., DALTON TRANS., 1989, pages 2115-2119, Londres, GB; G. HUNTER et al.: "Complexes of technetium, rhenium, and rhodium with sexidentate schiff-base ligands"<br>* Le article en entier *<br>--- | 1-9 | |
| Y | EP-A-0 299 795 (NYCOMED AS)<br>* Page 22, lignes 41-53; revendications *<br>--- -/- | 1-9 | |

| DOMAINES TECHNIQUES RECHERCHES (Int. Cl.5) |
|---|
| A 61 K |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 5

Revendications ayant fait l'objet de recherches incomplètes: 1-4,6-9

Revendications n'ayant pas fait l'objet de recherches:

Raison pour la limitation de la recherche:

voir feuille -C-

| Lieu de la recherche | Date d'achèvement de la recherche | Examinateur |
|---|---|---|
| LA HAYE | 04-03-1992 | SITCH W.D.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0408)

Page 2

Office européen des brevets

# RAPPORT PARTIEL DE RECHERCHE EUROPEENNE

Numero de la demande

EP 91 40 3291

| DOCUMENTS CONSIDERES COMME PERTINENTS | | | CLASSEMENT DE LA DEMANDE (Int. Cl.5) |
|---|---|---|---|
| Catégorie | Citation du document avec indication, en cas de besoin, des parties pertinentes | Revendication concernée | |
| Y | J. CHEM. SOC., DALTON TRANS., 1988, pages 2927-2933, Londres, GB; D.F. EVANS et al.: "Water-soluble hexadentate schiff-base ligands as sequestrating agents for iron(III) and gallium(III)<br>* Le article en entier *<br>--- | 1-9 | |
| D,A | AUST. J. CHEM., vol. 21, 1968, pages 1365-1367, Melbourne, AU; J.A. BROOMHEAD et al.: "A potential septadentate ligand"<br>* Le article en entier *<br>--- | | |
| D,A | EP-A-0 071 564 (SCHERING AG)<br>* Revendications *<br>----- | | DOMAINES TECHNIQUES RECHERCHES (Int. Cl.5) |

EPO FORM 1503 03.82 (P0411)

EP 91 40 3291

-C-

Les formules décrits dans les revendications 1,6 et 7 peuvent représenter un grand nombre de composés. Celles qui veulent être réellement protégées par les revendications ne sont pas suffisamment clairement délimitées (l'étendue souhaitée pour cette protection est trop floue).
Par conséquent, la recherche a du être limitée aux substances spécifiquement mentionnés dans la description et la revendication 5, de facon à réaliser une recherche significative et plus approfondie.